Europäisches Patentamt

European Patent Office

Office européen des brevets

(11) Veröffentlichungsnummer: **0 270 025 B1**

# EUROPÄISCHE PATENTSCHRIFT

(45) Veröffentlichungstag der Patentschrift: **27.01.93**

(51) Int. Cl.5: **A61K 39/395**

(21) Anmeldenummer: **87117566.7**

(22) Anmeldetag: **27.11.87**

(54) **Verfahren zur Herstellung eines intravenös anwendbaren und in flüssiger Form stabilen Immunglobulins.**

(30) Priorität: **02.12.86 DE 3641115**

(43) Veröffentlichungstag der Anmeldung:
**08.06.88 Patentblatt 88/23**

(45) Bekanntmachung des Hinweises auf die
Patenterteilung:
**27.01.93 Patentblatt 93/04**

(84) Benannte Vertragsstaaten:
**AT BE CH DE FR GB IT LI LU NL**

(56) Entgegenhaltungen:
**EP-A- 0 196 761**
**CA-A- 1 201 063**
**FR-A- 2 301 266**
**GB-A- 1 006 258**
**US-A- 4 351 710**

(73) Patentinhaber: **Heilmittelwerke Wien Gesellschaft m.b.H.**
**Doerenkampgasse 4**
**A-1100 Wien(AT)**

(72) Erfinder: **Doleschel, Walter, Univ.-Prof. Dr.**
**Sonnleitensteig 7**
**A-1190 Wien(AT)**
Erfinder: **Doleschel, Walter Norbert, Dr.**
**Siolygasse 18-22/3a**
**A-1190 Wien(AT)**
Erfinder: **Conrad, Werner, Dipl.-Ing.**
**Hebbelplatz 3/M3**
**A-1100 Wien(AT)**

(74) Vertreter: **Kunz, Ekkehard, Dr.**
**Chemie Holding AG Patentwesen St. Peter-Strasse 25**
**A-4021 Linz(AT)**

**Beschreibung**

Die vorliegende Erfindung betrifft ein verbessertes Verfahren zur Herstellung eines chemisch unmodifizierten, nicht enzymatisch behandelten, im wesentlichen Immunglobulin A (IgA)-freien Immunglobulin G (IgG), das frei von Nebenwirkungen ist, aus praktisch reinem IgG besteht und in Form einer stabilen klaren wässrigen Lösung oder als lyophilisiertes Präparat für die intravenöse Verabreichung zur Verfügung steht.

In der DE-OS 2606118 wird ein mehrstufiges Verfahren zur Herstellung eines intravenös verträglichen Gammaglobulin-Präparates beschrieben. Hierbei wird in einer Stufe a) eine Euglobulinfällung bei pH 4,8 bis 6,5 durchgeführt, in einer Stufe b) werden Verunreinigungen mit Polyethylenglykol (PEG) mit einem Molekulargewicht von 4 000 bei einer Konzentration von 4 und 5 % ausgefällt und in Stufe c) wird das IgG mit PEG 4 000 bei einer Konzentration von 12 % gefällt. In dieser Offenlegungsschrift werden jedoch keine Angaben über die Reinheit des Produktes, insbesondere über den Gehalt an Monomeren gemacht. Die nach diesem Verfahren erhaltenen Produkte haben jedoch den Nachteil, daß das als Fällungsmittel verwendete PEG im Präparat verbleibt. Ferner müssen zur Stabilisierung Albumin und ein oberflächenaktives Mittel zugesetzt werden. Letzteres ist jedoch vom medizinischen Standpunkt unerwünscht.

Aus der AT-PS 381 026 ist ein vielstufiges Verfahren zur Herstellung einer intravenös verabreichbaren und in flüssiger Form lagerstabilen IgG-Lösung bekannt, das jedoch in Bezug auf Ausbeute, Einfachheit des Verfahrens und auf Reinheit des Produktes nicht voll befriedigt, wobei auch die Entsorgung des Fällungsmittels in umweltfreundlicher Weise problematisch ist.

Es stellte sich daher die Aufgabe, ein einfacheres und gute Ausbeuten lieferndes Verfahren zur Herstellung eines intravenös verabreichbaren, in flüssiger Form lagerstabilen, oder lyophilisierten praktisch reinen Immunglobulins zu entwickeln.

Diese Aufgabe wird erfindungsgemäß dadurch gelöst, daß man eine vorgereinigte IgG-Lösung einer Kombination von mehreren aufeinander folgenden und aufeinander abgestimmten Behandlungsschritten unterwirft. In einer ersten Stufe werden unerwünschte Begleitproteine durch eine Euglobulinfällung beseitigt, anschließend folgt eine Säurestabilisierung und eine Behandlung mit einem Anionenaustauscher, durch den Immunglobuline, die nicht der IgG-Klasse angehören, z.B. IgA, wie auch andere Verunreinigungen und ein Teil der unphysiologischen IgG-Aggregate entfernt werden. In einem weiteren Schritt werden IgG-Aggregate und andere höhermolekulare Begleitproteine durch eine Fällung mit niedermolekularem Polyethylenglykol (PEG) gefällt und die überstehende reine IgG-Lösung durch Ultra- und Diafiltration in eine lagerstabile und injektionsfähige Form gebracht. Der Vorteil des niedermolekularen PEG gegenüber dem nach dem Stand der Technik verwendeten höhermolekularen PEG mit Molekulargewichten zwischen 4 000 und 6 000 besteht darin, daß sich niedermolekulares PEG durch Dialyse vollständig entfernen läßt, wobei es sich als besonders vorteilhaft erwiesen hat, wenn man die Entfernung des PEG mit der nach jeder Fraktionierung erforderlichen Konzentrierung der Proteinlösung kombiniert in einer Ultrafiltrationsanlage durchführt.

Gegenstand der vorliegenden Erfindung ist demnach ein verbessertes Verfahren zur Herstellung eines chemisch unmodifizierten, nicht enzymatisch behandelten, funktionell und strukturell intakten, nebenwirkungsfreien, alle IgG Subklassen enthaltenden, im wesentlichen IgA freien, in flüssiger Form lagerstabilen, intravenös verabreichbaren Immunglobulins mit einem Monomergehalt von über 97 % und einem Gehalt an Tri- und höheren Polymeren von unter 1 % durch Reinigung und Anreicherung einer nach bekannten Verfahren hergestellten Ausgangsfraktion von IgG in mehreren Schritten wie Dialysieren, Adsorbieren und Fällen, dadurch gekennzeichnet, daß man

a) eine wässrige Lösung der Ausgangsfraktion zur Entfernung niedermolekularer Bestandteile dialysiert, zur Entfernung der Euglobuline die Ionenstärke bei einem pH-Wert von 6,0 - 9,0 auf unter 500 $\mu$S erniedrigt, gegebenenfalls aus dem Überstand der Euglobulinfällung Verunreinigungen durch ein- oder zweimalige Adsorption an einen Anionenaustauscher in einer Menge von 5 - 50 mg/g Protein bei einem pH-Wert von 6,0 bis 9,0 entfernt,

b) die Lösung durch eine Säurebehandlung bei pH 2,5 - 4,2 stabilisiert, gegebenenfalls bei pH 3,0 - 8,0 für 15 bis 120 Minuten auf 40 - 60°C erhitzt, den pH-Wert auf 7,0 - 9,0 einstellt und die unlöslichen Anteile abzentrifugiert,

c) noch vorhandenes IgA und andere Verunreinigungen durch ein- oder zweimaliges Behandeln mit einem Anionenaustauscher in einer Menge von 5 - 50 mg/g Protein bei pH 6,0 - 9,0 entfernt,

d) die Lösung gegebenenfalls mit den im Endpuffer vorhandenen Substanzen versetzt, labile und aggregierte IgG-Moleküle durch Zugabe von Polyethylenglykol mit einem mittleren Molekulargewicht von 200 - 1 000 bis zu einer Endkonzentration von 10 - 25 % (w/w) ausfällt und abzentrifugiert, zu einer noch nicht mit den im Endpuffer vorhandenen Substanzen versetzten Lösung diese Substanzen zugibt, das Fällungsmittel Polyethylenglykol durch Dialyse oder Ultra- und Diafiltration entfernt, die Lösung auf die gewünschte Proteinkonzentration einstellt und sterilfiltriert.

Als Ausgangsmaterial für das erfindungsgemäße Verfahren kommen alle Fraktionen eines normalen oder hyperimmunen Plasmas und alle intramuskulär anwendbaren Präparate in Frage, die einen IgG-Gehalt zwischen 30 und 100 % des Gesamtproteins aufweisen. Solche Fraktionen können beispielsweise durch Ethanolfraktionierung nach Cohn als Fraktion II + III oder Fraktion II erhalten werden. Ähnliche Fraktionen können auch beispielsweise durch Ammoniumsulfatfällung, Polyethylenglykol- oder Rivanol-Fraktionierung hergestellt werden. Es können aber auch bereits fertige, für die intramuskuläre Anwendung bestimmte oder nicht in flüssiger Form lagerstabile, für die intravenöse Anwendung hergestellte Präparate dem erfindungsgemäßen Verfahren unterzogen werden.

Nach der AT-PS 381 026 werden als erste Reinigungsstufe zur Ausfällung der Euglobuline die Einstellung einer Leitfähigkeit von weniger als 300 $\mu$S bei einem pH Wert von 5.3 bis 5.5 mit anschließender Zugabe von BaSO$_4$ zur Adsorption von proteolytischen Enzymen vorgeschlagen. Überraschenderweise wurde nun festgestellt, daß die Adsorption an ein mineralisches Adsorbens wegfallen kann, wenn man die Euglobulinfällung in Stufe a) nach Entfernung niedermolekularer Bestandteile und eventuell vorhandener Fällungsmittelreste wie z.B. Ethanol durch Dialyse gegen aqua dest. bei einer Leitfähigkeit von unter 500 $\mu$S und einem pH Wert von 6.0 bis 9.0, bevorzugt 6,5 bis 7,5 vornimmt. Nach der Abtrennung des Euglobulinniederschlages kann, falls ein Ausgangsmaterial mit einem geringeren Reinheitsgrad verwendet wurde, eine ein- bis zweimalige Behandlung der klaren Lösung mit einem schwachen Anionenaustauscher erfolgen. Hiefür eignen sich alle zur Behandlung biologischer Substanzen hergestellten schwachen Anionenaustauscher wie z.B. DEAE-Sephadex®, DEAE-Sepharose, DEA Cellulofine u.ä. Bewährt hat sich hierbei die Verwendung von DEAE-Sephadex® A 50 in einer Menge von etwa 5,0 bis 50 mg, bevorzugt 10 - 30 mg Ionenaustauscher (Trockengewicht) pro Gramm Protein bei einem pH-Wert von 6,0 bis 9,0, bevorzugt pH 6,5 bis 7,5.

Die nach Stufe a) erhaltene Lösung wird zur Erhöhung der Stabilität und zur Verringerung der antikomplementären Aktivität in Stufe b) einer Säurebehandlung unterzogen. Diese kann in Abhängigkeit von der Dauer der Einwirkung bei einem pH-Wert von 2,5 bis 4,2, bevorzugt von 2,9 bis 3,9, erfolgen, wobei die Einstellung eines pH-Wertes von 3,3 bis 3,5 wiederum besonders bevorzugt ist. In einer günstigen Ausführungsform kann diese Behandlung in zwei Stufen durchgeführt werden, indem die Lösung zunächst mit 1N HCl bei 0 - 10°C, bevorzugt bei 4°C, auf einen pH-Wert von etwa 2,9 bis 3,1 gebracht und für 5 bis 10 Minuten bei diesem pH-Wert belassen wird. Anschließend wird durch Zugabe einer Base, bevorzugt von Ammoniak, ein pH-Wert von 3,3 bis 3,5 eingestellt und die klare Lösung für 12 - 72 Stunden, bevorzugt 12 bis 24 Stunden bei 0° - 10° C gehalten.

Durch weitere Zugabe einer Base wird der pH-Wert auf etwa 7,0 bis 9,0, bevorzugt pH 8,0 bis 8,5 eingestellt und 0,5 bis 5 Stunden, bevorzugt 1 - 3 Stunden, zur Ausflockung von Aggregaten und denaturierten Proteinen stehen gelassen. Gegebenenfalls kann man die Lösung auch, vor der Einstellung auf pH 7,0 bis 9,0 für etwa 15 Minuten bis 2 Stunden bei pH 3,0 bis 8,0 auf etwa 40 - 60°C, bevorzugt eine halbe bis eine Stunde auf 45 - 55°C erwärmen. Hiebei erhält man bei einer Hitzebehandlung im sauren Bereich ein besonders stabiles Produkt, während eine Hitzebehandlung im neutralen bis leicht alkalischen Bereich zu höheren Ausbeuten führt. Die Entfernung des Niederschlages kann dann auf übliche Art durch Filtrieren, Zentrifugieren oder Dekantieren erfolgen.

Wird ein Immunglobulin-Präparat gewünscht, das nicht in flüssiger Form vorliegen soll, so ist die Säure- und Temperaturbehandlung nicht unbedingt notwendig.

In einer Stufe c) wird der Überstand aus Stufe b) ein- bis zweimal mit einem schwachen Anionenaustauscher behandelt. Bewährt hat sich wiederum die Verwendung von DEAE-Sephadex® A 50 in einer Menge von etwa 5 - 50 mg (Trockengewicht) pro Gramm Protein bei einem pH-Wert von 6,0 bis 9,0, bevorzugt 10 - 30 mg, bei pH 7,0 bis 7,5. Die Einstellung eines neutralen bis leicht alkalischen pH-Wertes führt zu einem besonders IgA-armen IgG-Präparat.

Die Abtrennung von Dimeren und Aggregaten des IgG sowie von höhermolekularen Verunreinigungen und labilen Proteinen erfolgt im Schritt d) durch Zugabe von niedermolekularem Polyethylenglykol (PEG) mit einem mittleren Molekulargewicht von 200 - 1 000, wobei Molekulargewichte von 600 bis 1 000 bevorzugt sind. Die Endkonzentration an PEG zur Ausfällung der oben beschriebenen Bestandteile der IgG-Lösung hängt hierbei weitgehend vom Molekulargewicht des PEG ab und reicht von etwa 10 Gewichtsprozent bei PEG 1 000 bis etwa 25 Gewichtsprozent bei PEG 200. Der pH-Wert der Lösung soll während der PEG-Fällung etwa zwischen 6,0 und 8,0 liegen, bevorzugt zwischen 6,5 und 7,5 . Die Fällung wird vorteilhafterweise über Nacht bei 4° C gelagert, bevor der Niederschlag abzentrifugiert wird.

Die IgG-Lösung wird nun mit den im Endpuffer verwendeten Substanzen versetzt. Es können dazu alle physiologisch und mit IgG verträglichen Puffer verwendet werden, wie z.B. Phosphatpuffer, Glycin/Natriumchlorid u.ä. Dieser Schritt kann auch bereits vor der PEG-Fällung erfolgen. Die Entfernung des PEG kann nach mit in der Biochemie üblichen Methoden wie Umfällen, Dialysieren, Ultra-und Diafiltration erfolgen. Besonders bevorzugt ist hiebei jedoch, die klare Lösung in eine Ultrafiltrationsanlage zu überführen und dort durch Ultra- und Diafiltration gegen einen 5-fachen Überschuß des Endpuffers gleichzeitig die gewünschte Proteinkonzentration und den gewünschten Puffer einzustellen sowie das PEG aus der IgG-Lösung zu entfernen.

Anschließend wird die IgG-Lösung, die mindestens 97 % Monomere, meistens jedoch über 99 % Monomere enthält, sterilfiltriert und abgefüllt.

Bevorzugterweise wird das fertige Gammaglobulinpräparat in flüssiger und anwendungsfertiger Form gelagert, wobei es auch bei längerer Lagerung stabil bleibt. Es kann jedoch, falls erwünscht, auch lyophilisiert werden.

Beispiel 1:

25 g Cohn Niederschlag II-1,2 mit einem Proteingehalt von 25,6 % wurden mit 25 ml demineralisiertem Wasser versetzt und bis zum Vorliegen einer homogenen Suspension bei +4° C gerührt. Anschließend erfolgte eine Dialyse bei +4° C gegen fließendes, demineralisiertes Wasser. Nach 48 Stunden wurde mit der 4-fachen Menge demineralisiertem Wasser verdünnt und eine Natriumchloridkonzentration von 1 mmol/l eingestellt. Die Lösung wurde zentrifugiert und der Niederschlag, der einen hohen Anteil an höhermolekularen Proteinen enthielt, wurde verworfen.

Der Überstand wurde mit 20 mg DEAE-Sephadex A 50 (Trockengewicht) pro Gramm Protein versetzt und 2 Stunden bei +4° C gerührt. Der Ionenaustauscher wurde über eine Nutsche abgesaugt, der pH auf 7,0 nachgestellt und die Behandlung mit DEAE-Sephadex A 50 wiederholt.

Durch Zugabe von 1N HCl wurde der pH der Proteinlösung auf 2,9 eingestellt, nach 10 Minuten rühren wurde durch Zugabe von Ammoniaklösung ein pH von 3,3 eingestellt und die Lösung über Nacht bei +4° C gelagert. Durch weitere Zugabe von Ammoniaklösung wurde der pH auf 8,25 erhöht, die Lösung 2 Stunden zur Ausflockung von Aggregaten bei +4° C gelagert und der entstandene Niederschlag abzentrifugiert und verworfen.

Zur Entfernung geringer Reste von labilen IgG-Molekülen und proteolytischen Enzymen wurde nochmals mit Anionenaustauscher (20 mg/g Protein) 2 Stunden bei +4° C gerührt und anschließend abgesaugt.

Um einen Reinheitsgehalt der Endlösung an monomerem Immunglobulin G zu sichern, der größer als 97 %, in der Regel jedoch größer als 99 % ist, erfolgte eine Fällung durch Zugabe von PEG 1 000 (als 50 %-ige wäßrige Lösung) auf einen Gehalt von 10 % (w/w). Die Fällung wurde über Nacht bei +4 ° C gelagert, bevor der Niederschlag abzentrifugiert wurde.

Die reine IgG-Lösung wurde anschließend durch Zugabe von Glycin (15 g/l) und NaCl (7 g/l) stabilisiert und durch Ultra- bzw. Diafiltration durch eine Millipore Cassette PTGC 00005 mit einer Molekulargewichtsausschlußgrenze von 10 000 gegen eine fünffache Menge an Glycin-NaCl-Puffer vom PEG 1000 befreit, wobei gleichzeitig die Proteinkonzentration auf 49,89 mg/ml erhöht wurde. Anschließend wurde die Lösung sterilfiltriert und abgefüllt.

Ausbeute: 98 ml

Antikomplementäre Aktivität: 16,9

(Die antikomplementäre Aktivität wurde nach KABAT, E.A. und MAYER, M.M.: Experimental Immunochemistry, 2nd Ed., Charles C. Thomas, p. 135 - 153, 1961 bestimmt.)

| | % Protein | % Monomere | % Dimere | % Polymere | IgA-Titer |
|---|---|---|---|---|---|
| aufge-löster NS II-1,2 | 100 | 88,5 | 8,0 | 3,5 | 1:8 |
| Überstand nach Dialyse | 91,1 | 92,7 | 5,8 | 1,5 | 1:8 |
| Überstand nach 1. DEAE-Beh. | 85,2 | 97,4 | 1,9 | 0,7 | 0 |
| Überstand nach 2. DEAE-Beh. | 83,3 | 97,9 | 1,5 | 0,6 | 0 |
| nach Säure-stabilis. | 82,8 | 97,8 | 1,6 | 0,6 | 0 |
| Überstand nach 3. DEAE-Beh. | 79,7 | 98,6 | 1,1 | 0,3 | 0 |
| Überstand nach PEG 1 000 | 77,2 | 99,1 | 0,9 | 0 | 0 |
| nach Dialyse | 76,4 | 99,1 | 0,9 | 0 | 0 |

Tabelle 1:

Der Proteingehalt der Lösung, die nach Auflösen des Niederschlages II-1,2 erhalten wurde, wurde gleich 100 % gesetzt.

Der Gehalt an IgA wurde mittels Ouchterlonytechnik bestimmt.

Die Verteilung der Mono-, Di- und Polymeren wurde mittels HPLC (LKB, Säule TSK G 3000 SW 7,5 × 600 mm) bestimmt.

Beispiel 2:

49,65 g Cohn Niederschlag II-1,2 mit einem Proteingehalt von 26,2 % wurden in 50 ml demineralisiertem Wasser gelöst und 2 Tage gegen fließendes demineralisiertes Wasser dialysiert. Nach Zugabe von 400 ml demineralisiertem Wasser wurde die Lösung zentrifugiert.

Der Überstand wurde durch Zugabe von 1 N HCl auf einen pH von 2,92 eingestellt, nach 10 Minuten Rühren wurde auf einen pH von 3,28 durch Zugabe der nötigen Menge 1 N NaOH zurückgestellt. Die Proteinlösung wurde über Nacht bei +4 ° C gelagert und danach auf einen pH von 8,3 durch weitere Zugabe von 1 N NaOH eingestellt, um eine optimale Ausfällung von labilen Proteinen zu erreichen. Der Niederschlag wurde abzentrifugiert und verworfen.

Nach Zugabe des gleichen Volumens demineralisierten Wassers wurden 20 mg DEAE-Sephadex A50 pro Gramm Protein zugegeben. Es wurde eine Stunde bei +4° C gerührt. Der Ionenaustauscher wurde durch Filtration entfernt, und der pH der Proteinlösung auf 7,0 eingestellt.

Die Rührung mit 20 mg DEAE-Sephadex A50 pro Gramm Protein über 1 Stunde bei +4 ° C wurde wiederholt, anschließend wurde der Ionenaustauscher durch Filtration entfernt. Die Stabilisierung der Immunglobulinlösung erfolgte durch Zugabe von Glycin (15 g/l) und Natriumchlorid (7 g/l), der pH-Wert wurde auf 7,0 eingestellt.

Anschließend erfolgte eine Fällung durch Zugabe von PEG 600 bis zu einer Konzentration von 15 % (w/w). Nach Lagerung dieser Fällung über Nacht bei +4° C wurde der Niederschlag abzentrifugiert.

Die Abtrennung von PEG 600 und die Konzentrierung auf einen Proteingehalt von 50,27 mg/ml erfolgte entsprechend Beispiel 1.
Ausbeute: 199 ml
Antikomplementäre Aktivität: 18,2

| | % Protein | % Monomere | % Dimere | % Polymere | IgA-Titer |
|---|---|---|---|---|---|
| aufgelöster Cohn NS II-1,2 | 100 | 88,5 | 8,1 | 3,4 | 1:8 |
| Überstand nach Dialyse | 89,9 | 93,4 | 5,2 | 1,4 | 1:8 |
| nach Säure- stabilisierung | 89,1 | 93,0 | 5,3 | 1,7 | 1:8 |
| Überst. nach 1.DEAE-Beh. | 82,9 | 97,0 | 2,2 | 0,8 | 0 |
| Überst.nach 2.DEAE-Beh. | 80,2 | 98,3 | 1,3 | 0,4 | 0 |
| Überst.nach PEG 600 | 78,0 | 99,4 | 0,6 | 0 | 0 |
| nach Dialyse | 76,9 | 99,4 | 0,6 | 0 | 0 |

Tabelle 2:

Der Proteingehalt der Lösung, die nach Auflösen des Niederschlages II-1,2 erhalten wurde, wurde gleich 100 % gesetzt.
Der Gehalt an IgA wurde mittels Ouchterlonytechnik bestimmt.
Die Verteilung der Mono-, Di- und Polymeren wurde mittels HPLC (LKB, Säule TSK G 3 000 SW 7,5 × 600 mm) bestimmt.

Beispiel 3:

30 g Cohn Niederschlag II-1,2 mit einem Proteingehalt von 28 % wurden mit 30 ml demineralisiertem Wasser bei 4° C angerührt. Die anschließende Euglobulinfällung wurde analog Beispiel 1 durchgeführt. Der Überstand wurde mit 25 mg DEAE-Sephadex A50/g Protein versetzt, der pH auf 7,0 korrigiert, die Suspension 2 Stunden bei 4° C gerührt und der Ionenaustauscher durch Filtration über einen Buchnertrichter entfernt.

Das Filtrat wurde durch Zugabe von 1 n HCl auf eine pH von 2,9 eingestellt. Nach 10 minütigem Rühren wurde durch Zugabe von Ammoniaklösung ein pH von 3,3 eingestellt und die Lösung über Nacht bei +4° C gelagert.

Durch Zugabe von Ammoniaklösung wurde der pH auf 7,5 erhöht, die Lösung auf 45° C erwärmt und 30 Minuten bei dieser Temperatur gehalten. Nach dem Abkühlen wurden die denaturierten Proteine abzentrifugiert.

Der Überstand wurde nochmals mit 25 mg DEAE-Sephadex A50/g Protein versetzt, der pH-Wert auf 7,0 nachgestellt, die Lösung 2 Stunden bei 4° C gerührt und der Ionenaustauscher abfiltriert.

Durch Zugabe von PEG 1000 als 50 %-ige wäßrige Lösung bis auf eine Konzentration von 10 % (w/w) und Lagerung über Nacht bei +4° C erfolgte eine weitere Ausfällung von höhermolekularen Verunreinigungen bzw. oligomeren Formen von IgG.

Nach der Zentrifugation wurde die reine IgG-Lösung durch Zugabe von Glycin (15g/l) und NaCl (7g/l) stabilisiert. Durch Ultra- und Diafiltration entsprechend Beispiel 1 wurde das PEG 1 000 entfernt und die IgG-Lösung auf 50,26 mg Protein pro ml konzentriert.
Ausbeute: 124 ml
Antikomplementäre Aktivität: 19,8

|  | % Protein | % Monomere | % Dimere | % Polymere | IgA-Titer |
|---|---|---|---|---|---|
| aufgelöster | | | | | |
| NS II-1,2 | 100 | 88,3 | 8,3 | 3,4 | 1:8 |
| Überst.nach | | | | | |
| PEG 1000 | 75,4 | 99,6 | 0,4 | 0 | 0 |
| nach Dialyse | 74,2 | 99,5 | 0,5 | 0 | 0 |

## Tabelle 3:

Der Proteingehalt der Lösung, die nach Auflösen des Niederschlages II-1,2 erhalten wurde, wurde gleich 100 % gesetzt.
Der Gehalt an IgA wurde mittels Ouchterlonytechnik bestimmt. Die Verteilung der Mono-, Di- und Polymeren wurde mittels HPLC (LKB, Säule TSK G 3 000 SW 7,5 × 600 mm) bestimmt.

Beispiel 4:

Herstellung einer gefriergetrockneten Präparation.

50,1 g Cohn Niederschlag II-1,2 mit einem Proteingehalt von 29,6 % wurde in 50 ml demineralisiertem Wasser gelöst und 2 Tage gegen fließendes Leitungswasser und einen Tag gegen demineralisiertes Wasser dialysiert. Nach Zugabe von 400 ml demineralisiertem Wasser wurde die stark trübe Lösung mit NaCl auf eine Konzentration von 1 Mmol/1 eingestellt, der pH auf 7,0 korrigiert und abzentrifugiert.
Der Überstand wurde mit 50 mg DEAE-Sephadex A50 pro Gramm Protein versetzt und bei +4° C 2 Stunden gerührt. Der Ionenaustauscher wurde durch Filtration über einen Buchnertrichter abgetrennt.
Die Immunglobulinlösung wurde auf einen Gehalt von 15 g/l Glycin und 7 g/l NaCl eingestellt. Durch Zugabe von PEG 1000 (als 50 %-ige wäßrige Lösung) auf eine Konzentration von 10 % (w/w) wurden labile und leicht aggregierbare Proteine ausgefällt, wobei die Fällung über Nacht bei +4° C gelagert wurde. Danach wurde der Niederschlag durch Zentrifugation entfernt.
Durch Ultra- bzw. Diafiltration wurde die Proteinkonzentration auf 50,03 g/l erhöht und das PEG 1000 entfernt. Nach einer Klär- und Sterilfiltration wurde die Immunglobulin G-Lösung abgefüllt und gefriergetrocknet.
Ausbeute: 222 ml
Antikomplementäre Aktivität: 20,9

7

|  | % Protein | % Monomere | % Dimere | % Polymere | IgA-Titer |
|---|---|---|---|---|---|
| aufgelöster | | | | | |
| NS-1,2 | 100 | 87,4 | 8,5 | 4,1 | 1:16 |
| Überstand | | | | | |
| nach Dialyse | 90,2 | 92,7 | 5,7 | 1,6 | 1:8 |
| Überst. nach | | | | | |
| DEAE-Beh. | 83,0 | 96,6 | 2,5 | 0,9 | 0 |
| Überst. nach | | | | | |
| PEG 1000 | 78,4 | 98,8 | 1,2 | 0 | 0 |
| nach Dialyse | 74,9 | 98,7 | 1,3 | 0 | 0 |

Tabelle 4:

Der Proteingehalt der Lösung, die nach Auflösen des Niederschlages II-1,2 erhalten wurde, wurde gleich 100 % gesetzt.
Der Gehalt an IgA wurde mittels Ouchterlonytechnik bestimmt.
Die Verteilung der Mono-, Di- und Polymeren wurde mittels HPLC (LKB, Säule TSK G 3000 SW 7,5 × 600 mm) bestimmt.

**Patentansprüche**

1. Verfahren zur Herstellung eines chemisch unmodifizierten, nicht enzymatisch behandelten, funktionell und strukturell intakten, nebenwirkungsfreien, alle IgG Subklassen enthaltenden, im wesentlichen IgA freien, in flüssiger Form lagerstabilen, intravenös verabreichbaren Immunglobulins mit einem Monomergehalt von über 97 % und einem Gehalt an Tri- und höheren Polymeren von unter 1 % durch Reinigung und Anreicherung einer nach bekannten Verfahren hergestellten Ausgangsfraktion von IgG in mehreren Schritten wie Dialysieren, Adsorbieren und Fällen, dadurch gekennzeichnet, daß man
    a) eine wässrige Lösung der Ausgangsfraktion zur Entfernung niedermolekularer Bestandteile dialysiert, zur Entfernung der Euglobuline die Ionenstärke bei einem pH-Wert von 6,0 - 9,0 auf unter 500 μS erniedrigt, gegebenenfalls aus dem Überstand der Euglobulinfällung Verunreinigungen durch ein- oder zweimalige Adsorption an einen Anionenaustauscher in einer Menge von 5 - 50 mg/g Protein bei einem pH-Wert von 6,0 bis 9,0 entfernt,
    b) die Lösung durch eine Säurebehandlung bei pH 2,5 - 4,2 stabilisiert, gegebenenfalls bei pH 3,0 - 8,0 für 15 bis 120 Minuten auf 40 - 60° C erhitzt, den pH-Wert auf 7,0 - 9,0 einstellt und die unlöslichen Anteile abzentrifugiert,
    c) noch vorhandenes IgA und andere Verunreinigungen durch ein- oder zweimaliges Behandeln mit einem Anionenaustauscher in einer Menge von 5 - 50 mg/g Protein bei pH 6,0 - 9,0 entfernt,
    d) die Lösung gegebenenfalls mit den im Endpuffer vorhandenen Substanzen versetzt, labile und aggregierte IgG-Moleküle durch Zugabe von Polyethylenglykol mit einem mittleren Molekulargewicht von 200 - 1 000 bis zu einer Endkonzentration von 10 - 25 % (w/w) ausfällt und abzentrifugiert, zu einer noch nicht mit den im Endpuffer vorhandenen Substanzen versetzten Lösung diese Substanzen zugibt, das Fällungsmittel Polyethylenglykol durch Dialyse oder Ultra- und Diafiltration entfernt, die Lösung auf die gewünschte Proteinkonzentration einstellt und sterilfiltriert.

2. Verfahren nach Anspruch 1, dadurch gekennzeichnet, daß man das nach Anspruch 1 ohne die Stufe b)

8

erhaltene Immunglobulin lyophilisiert.

3. Verfahren nach einem der Ansprüche 1 bis 2, dadurch gekennzeichnet, daß die Ausgangsfraktion 30 - 100 % IgG enthält.

4. Verfahren nach einem der Ansprüche 1 bis 3, dadurch gekennzeichnet, daß man in Stufe a) die Euglobulinfällung bei einem pH-Wert von 6,5 bis 7,5 vornimmt.

5. Verfahren nach einem der Ansprüche 1 bis 4, dadurch gekennzeichnet, daß man in Stufe a) die Adsorption mit 10 - 30 mg Anionenaustauscher pro g Protein bei pH 6,5 bis 7,5 vornimmt.

6. Verfahren nach einem der Ansprüche 1 bis 5, dadurch gekennzeichnet, daß die Säurebehandlung in Stufe b) in 2 Schritten durchgeführt wird, indem durch Ansäuern für 5 - 10 min. bei 4° C ein pH-Wert von 2,9 bis 3,1 eingestellt und anschließend die Lösung nach Rückstellung des pH-Wertes auf 3,3 bis 3,5 für 12 bis 24 Stunden gelagert wird.

7. Verfahren nach einem der Ansprüche 1 bis 6, dadurch gekennzeichnet, daß man die Temperaturbehandlung in Stufe b) bei pH 3,0 bis 8,0 bei 45 bis 55° C für 30 bis 60 min. durchführt.

8. Verfahren nach einem der Ansprüche 1 bis 7, dadurch gekennzeichnet, daß man die Behandlung mit einem Ionenaustauscher in Stufe c) bei pH 7,0 bis 7,5 in einer Menge von 10 - 30 mg Ionenaustauscher pro Gramm Protein durchführt.

9. Verfahren nach einem der Ansprüche 1 bis 8, dadurch gekennzeichnet, daß man die Fällung in Stufe d) mit einem PEG eines mittleren Molekulargewichtes von 600 - 1000 bis zu einer Endkonzentration von 10 - 15 % (w/w) bei pH 6,5 - 7,5 durchführt.

10. Verfahren nach einem der Ansprüche 1 bis 9, dadurch gekennzeichnet, daß man das als Fällungsmittel verwendete niedermolekulare PEG durch Ultra-und Diafiltration entfernt, gleichzeitig das IgG in den gewünschten Endpuffer überführt und gleichzeitig die gewünschte Proteinkonzentration einstellt.

## Claims

1. Process for the preparation of a chemically unmodified, functionally and structurally intact immunoglobulin which has not been enzymatically treated, has no side effects, contains all IgG subclasses, is essentially free of IgA, is stable on storage in liquid form and can be administered intravenously, and which has a monomer content of above 97% and a content of trimers and higher polymers of below 1%, by subjecting a starting fraction of IgG which has been prepared by known processes to purification and concentration in several step, such as dialysis, adsorption and precipitation, characterised in that

a) an aqueous solution of the starting fraction is dialysed to remove low molecular weight constituents, the ionic strength at a pH of 6.0 - 9.0 is lowered to below 500 $\mu$S in order to remove euglobulins, where appropriate impurities are removed from the supernatant from the euglobulin precipitation by adsorption once or twice on an anion exchanger in an amount of 5 - 50 mg/g protein at a pH of 6.0 to 9.0,

b) the solution is stabilised by acid treatment at pH 2.5 - 4.2, where appropriate heated at 40 - 60° C and pH 3.0 - 8.0 for 15 to 120 minutes, the pH is adjusted to 7.0 - 9.0, and the insoluble fractions are removed by centrifugation,

c) IgA and other impurities which are still present are removed by treatment once or twice with an anion exchanger in an amount of 5 - 50 mg/g protein at pH 6.0 - 9.0,

d) where appropriate, the substances present in the final buffer are added to the solution, labile and aggregated IgG molecules are precipitated by addition of polyethylene glycol having a mean molecular weight of 200 - 1,000 to a final concentration of 10 - 25% (w/w) and are removed by centrifugation, the substances present in the final buffer are added to a solution which does not yet contain these substances, the precipitant polyethylene glycol is removed by dialysis or ultra- and diafiltration, the solution is adjusted to the desired protein concentration and is sterilised by filtration.

2. Process according to Claim 1, characterised in that the immunoglobulin obtained according to Claim 1 without stage b) is freeze-dried.

3. Process according to one of Claims 1 to 2, characterised in that the starting fraction contains 30 - 100% IgG.

4. Process according to one of Claims 1 to 3, characterised in that the euglobulin precipitation in stage a) is carried out at a pH of 6.5 to 7.5.

5. Process according to one of Claims 1 to 4, characterised in that the adsorption in stage a) is carried out with 10 - 30 mg of anion exchanger per g of protein at pH 6.5 to 7.5.

6. Process according to one of Claims 1 to 5, characterised in that the acid treatment in stage b) is carried out in two steps by a pH of 2.9 to 3.1 being adjusted by acidification at 4°C for 5 - 10 min, and then the solution being readjusted to a pH of 3.3 to 3.5 and stored for 12 to 24 hours.

7. Process according to one of Claims 1 to 6, characterised in that the heat treatment in stage b) is carried out at pH 3.0 to 8.0 and at 45 to 55°C for 30 to 60 min.

8. Process according to one of Claims 1 to 7, characterised in that the treatment with an ion exchanger in stage c) is carried out at pH 7.0 to 7.5 in an amount of 10 - 30 mg of ion exchanger per gram of protein.

9. Process according to one of Claims 1 to 8, characterised in that the precipitation in stage d) is carried out with a PEG of a mean molecular weight of 600 - 1,000 to a final concentration of 10 - 15% (w/w) at pH 6.5 - 7.5.

10. Process according to one of Claims 1 to 9, characterised in that the low molecular weight PEG used as precipitant is removed by ultra- and diafiltration, the IgG is simultaneously transferred into the desired final buffer, and the desired protein concentration is simultaneously adjusted.

**Revendications**

1. Procédé pour préparer une immunoglobuline chimiquement non modifiée, traitée de manière non enzymatique, intacte fonctionnellement et structurellement, sans effets secondaires, renfermant toutes les sous-classes d'IgG exempte pour l'essentiel d'IgA, stockable sous forme liquide, administrable par intraveineuse, ayant une proportion en monomère supérieure à 97 % et une teneur en polymères triples et polymères supérieurs inférieure à 1 %, par purification et enrichissement d'une fraction d'IgG de départ obtenue selon des procédés connus, en plusieurs étapes telles les dialyses, les adsorbtions et précipitations, caractérisé en ce que :

(a) on soumet à dialyse une solution aqueuse d'une fraction de départ pour éliminer les constituants à faible poids moléculaire, on abaisse l'acidité pour éliminer les euglobulines à un pH de 6,0 à 9,0 en-dessous de 500 $\mu$S, on élimine le cas échéant du surplus de précipitation d'euglobuline les impuretés par une adsorption simple ou double sur un échangeur d'anions selon une quantité de 5 à 50 mg/g de protéine à un pH de 6,0 à 9,0,

(b) on stabilise la solution par un traitement acide à un pH de 2,5 à 4,2, on chauffe le cas échéant à une température de 40 à 60°C à un pH de 3,0 à 8,0 pendant 15 à 120 minutes, on ajuste le pH à 7,0 - 9,0, et on élimine par centrifugation les parties non dissoutes,

(c) on élimine les IgA encore présentes et les autres impuretés par un traitement simple ou double à l'aide d'un échangeur d'anions selon une quantité de 5 à 50 mg par gramme de protéine à un pH de 6,0 à 9,0,

(d) on mélange la solution le cas échéant avec les substances présentes dans la solution tampon finale, on précipite et on sépare par centrifugation les molécules d'IgG labiles et agrégées par addition de polyéthylèneglycol ayant une masse moléculaire moyenne de 200 à 1000 jusqu'à une concentration finale de 10 à 25 %, on ajoute ces substances à une solution non encore additionnée des substances présentes dans la solution tamponnée finale, on élimine le moyen de précipitation, le polyéthylèneglycol, par dialyse ou ultrafiltration et diafiltration, on ajuste la solution à la concentration en protéine souhaitée et on filtre de manière stérile.

**2.** Procédé selon la revendication 1, caractérisé en ce qu'on lyophilise l'immunoglobuline obtenue selon la revendication 1 sans mettre en oeuvre l'étape (b).

**3.** Procédé selon l'une des revendications 1 ou 2, caractérisé en ce que la fraction de départ renferme 30 à 100 % d'IgG.

**4.** Procédé selon l'une des revendications 1 à 3, caractérisé en ce qu'on entreprend dans l'étape (a) la précipitation de l'euglobuline à un pH compris entre 6,5 et 7,5.

**5.** Procédé selon l'une des revendications 1 à 4, caractérisé en ce qu'on entreprend dans l'étape (a) l'adsorption avec 10 à 30 mg d'échangeur d'anions par gramme de protéine à un pH de 6,5 à 7,5.

**6.** Procédé selon l'une des revendications 1 à 5, caractérisé en ce qu'on réalise le traitement acide de l'étape (b) en deux étapes, selon lequel on ajuste le pH à une valeur de 2,9 à 3,1 en acidifiant pendant 5 à 10 minutes à 4°C, et ensuite on laisse reposer la solution pendant 12 à 24 heures après avoir amené le pH à une valeur de 3,3 à 3,5.

**7.** Procédé selon l'une des revendications 1 à 6, caractérisé en ce qu'on réalise le traitement par chauffage de l'étape (b) à un pH de 3,0 à 8,0 à une température de 45 à 55°C pendant 30 à 60 minutes.

**8.** Procédé selon l'une des revendications 1 à 7, caractérisé en ce qu'on réalise le traitement des résines échangeuses d'ions de l'étape (c) à un pH compris entre 7,0 et 7,5 en utilisant une quantité comprise entre 10 et 30 mg de résine échangeuse d'ions par gramme de protéine.

**9.** Procédé selon l'une des revendications 1 à 8, caractérisé en ce qu'on conduit la précipitation de l'étape (d) avec un PEG ayant une masse moléculaire moyenne de 600 à 1000 jusqu'à une concentration finale de 10 à 15 % à un pH compris entre 6,5 et 7,5.

**10.** Procédé selon l'une des revendications 1 à 9, caractérisé en ce qu'on élimine le moyen de précipitation, le PEG à bas poids moléculaire utilisé, par ultrafiltration et diafiltration, on transfère en même temps l'IgG dans le tampon final désiré, et on ajuste en même temps la concentration en protéines souhaitée.